# EUROPEAN PATENT APPLICATION

(11) **EP 2 335 746 A1**
(43) Date of publication of application: **22.06.2011**
(21) Application number: 09816178.9
(22) Date of filing: 24.09.2009
(51) Int. Cl.: A61L 31/00, C08L 101/16

(54) **MEDICAL DEVICE, MEDICAL MATERIAL AND METHODS FOR PRODUCING SAME**

(30) Priority: 29.09.2008 JP 2008251390
(71) Applicant: Terumo Kabushiki Kaisha, Tokyo 151-0072 (JP)
(72) Inventor: KOHAMA Hiromasa, Ashigarakami-gun Kanagawa 259-0151 (JP); KIMINAMI Hideaki, Ashigarakami-gun Kanagawa 259-0151 (JP)
(74) Representative: TBK
(86) International application number: PCT/JP2009/066565
(87) International publication number: WO 2010/035763

(57) **Abstract**

A medical device and medical material using an aromatic biodegradable resin composition, which is not susceptible to decrease in strength and impact resistance during sterilization by ionizing radiation. Specifically disclosed are ionizing radiation sterilized medical device and medical material, which are **characterized by** containing a composition composed of an aromatic polyester-based biodegradable resin and a polylactic acid, with the polylactic acid being 25 to 70 wt% of the weight of the composition. The medical device and medical material are biodegradable, while having excellent strength and impact resistance.

## Description

### Technical Field

This invention relates to a medical device and medical material made of a biodegradable resin. The medical device and medical material of the invention are suitably used, for example, in medical fields. The invention also relates belongs to a method for making a medical device and medical material, made of a biodegradable resin.

### Background Art

Medical devices and medical materials can be employed in environments of body fluids, such as blood, urine and the like. In order to prevent infection with viruses, bacteria and the like medical devices and medical materials can be provided as a plastic, single-use, disposable article, and waste products thereof can be carefully disposed of such as by incineration. However, such disposal deeds lead to an increasing amount of waste, thereby causing an amount of carbon dioxide exhausted by the incineration disposal to be increased.

On the other hand, biodegradable resins are decomposed into carbon dioxide and water by microorganisms occurring in nature after completion of the life cycle thereof. Therefore, they have been expected to expand utility in many fields such as of agricultural materials, civil engineering and construction materials, industrial materials and the like for use as a material having a reduced load on environment. Among them, biodegradable resins prepared from plant-derived materials have a feature in that since carbon dioxide emitted upon disposal corresponds to an absorbed one at the stage of plant growth, there occurs no change in amount of carbon dioxide as a whole (carbon neutral) and thus, attention has been drawn thereto from the standpoint of stopping global warming.
Examples of the plant-derived biodegradable resins include those having flexibility such as polybutylene adipate/terephthalate copolymers and polybutylene succinate, and relatively hard ones such as polylactic acid and copolymers, blends and polymer alloys thereof. Although polybutylene/terephthalate copolymers and polybutylene succinate are now prepared from petroleum-derived materials, it has been planned to convert part of the starting materials to a plant-derived one.

The use of these biodegradable resins as a resin used in medical devices and medical materials that usually allow for disposal leads to the reduction of load on environment and is thus very effective. However, these biodegradable resins are lower in heat resistance, mechanical strength and molding processability than general-purpose resins such as polyethylene and polypropylene. Hence, in order to develop full-fledged applications, the resin design and improvements of physical properties such as by addition of modifiers can be important. In many medical devices, ionizing radiation sterilization has been carried out for its simplicity. Since biodegradable resins are more susceptible to lowering of strength and impact resistance than the above-mentioned general-purpose resins when subjected to ionizing radiation irradiation, a difficulty remains in their application to medical devices.
Hitherto, polylactic acid has had a problem in that its impact resistance lowers by application of ionizing radiation irradiation. With resins blended with polylactic acid, the lowering of impact resistance becomes prominent, so that it has not been possible to blend polylactic acid so as to upgrade a flexible resin such as a polybutylene succinate.
The polybutylene adipate/terephthalate copolymer is a flexible resin like polyethylene and is excellent in impact resistance and is thus suited as a softening agent for resin. In addition, rigidity can be increased by blending or alloying of a polybutylene adipate/terephthalate copolymer with polylactic acid and thus, the material can be designed for use as a resin for medical device member while meeting requirements therefor. However, there have never been known any examples under scrutiny as to whether blending or alloying of polybutylene adipate/terephthalate with polylactic acid suppresses lowering of strength and impact resistance when the blend or alloy is subjected to ionizing radiation sterilization.

With respect to the moldings of biodegradable resins capable of ionizing radiation sterilization, there is disclosed a technique wherein ionizing radiation sterilization is carried out after addition of a radiation crosslinking agent to a biodegradable resin (see, for example, Patent Document 1). Additionally, there is disclosed a technique wherein a polycarbodiimide serving as a terminal stopping agent is added for improving a hydrolysis resistance of biodegradable resin (see, for example, Patent Document 2).
However, it has never been known whether strength and impact resistance are suppressed from lowing after ionizing radiation sterilization by blending an aromatic polyester-based biodegradable resin, such as a polybutylene adipate/terephthalate copolymer, with polylactic acid.

### Prior Art Document

### Patent Document

Patent Document 1: Japanese Laid-open Patent Application No. 2004-204195
Patent Document 2: Japanese Patent No. 3776578

### Summary of Invention

### Technical Problem

The invention has been made in view of such problems and has for its object the provision of a medical device and medical material making use of an aromatic polyester-based biodegradable resin composition that is less susceptible to lowering of strength and impact resistance when subjected to sterilization by irradiation with an ionizing radiation.

### Technical Solution

The present investors made intensive studies and, as a result, found that when a biodegradable resin containing a polybutylene adipate/terephthalate copolymer in polylactic acid is irradiated with an ionizing radiation at a dose employed for sterilization of medical devices, the lowering of strength and impact resistance is reduced, thereby arriving at completion of the invention.

Such an object can be achieved by the inventions of (1) to (4) indicated below.
(1) An ionizing radiation-sterilized medical device and medical material, characterized by including a composition made of an aromatic polyester-based resin and polylactic acid wherein 25 to 70 wt% of the composition is polylactic acid.

(2) The medical device and medical material as recited in (1) above, wherein the aromatic polyester-based biodegradable resin is made of a polybutylene adipate/terephthalate copolymer, a polybutylene succinate/terephthalate copolymer or a copolymer thereof with polylactic acid.

(3) A method for making a medical device and medical material, characterized by including molding a composition made of an aromatic polyester-based biodegradable resin and polylactic acid wherein 25 to 70 wt% of the composition is polylactic acid, and subjecting to irradiation with an ionizing radiation.

(4) The method for making a medical device and medical material as recited in (3) above, wherein the aromatic polyester-based biodegradable resin is preferably made of a polybutylene adipate/terephthalate copolymer, a polybutylene succinate/terephthalate copolymer or a copolymer thereof with polylactic acid.

### Effect of the Invention

Because strength and an impact resistance prior to sterilization can be sustained without being substantially damaged at the stage of irradiation with an ionizing radiation by addition of a polybutylene adipate/terephthalate copolymer to polylactic acid, it has become possible to provide an ionizing radiation-sterilized medical device and medical material that are biodegradable and are excellent in strength and resistant to impact. Especially, changes of strength and impact resistance prior to and after irradiation are quite small, with relatively high general versatility.

### Best Mode for Carrying Out the Invention

The medical device and medical material of the invention are now described in detail.
The medical device used herein can include a mechanical device used in operations, treatments and diagnosis of the human or animal body and are more particularly those within ranges appearing in class ten of the Appended Table of the Ordinance for Enforcement of the Trademark Act (Ordinance of the Ministry of Economy, Trade and Industry No. 202 of Heisei 13 (2001)). Additionally, the medical material can include one which is used for distribution and employment of medicinal drugs and medical devices, e.g. a packaging material for medicinal drug and medical device and the accompanying items therefor and which is discarded after use of the medicinal drug and medical device. The medicinal drug can include a drug used for operation, treatment or diagnosis of the human or animal body and particularly includes ones within ranges appearing in class 5 of the appended table.

The ionizing radiation used in the present invention indicates an electromagnetic wave or particle beam (beam) having ionizability and a high energy except for a low energy radiation having no ionization effect. Hereinafter, it is referred to simply as radiation.
The strength used herein refers to a yield point stress in a tensile test and the impact resistance refer to an elongation at breakage in the tensile test.

The medical device and medical material (hereinafter referred to as medical device, etc.) of the invention are characterized in that they are constituted substantially of biodegradable resins and are made of a composition containing polylactic acid and an aromatic polyester-based biodegradable resin and that the effect of sustaining strength and an impact resistance of the resin composition at the time of irradiation of a radiation is great. Preferably, the medical device, etc. of the invention have parts creating the mechanical structures thereof (a chassis, a cover, a container, a casing and the like) or a device entirety, which is made of a composition composed substantially of a mixture of polylactic acid and an aromatic polyester-based biodegradable resin. The term "substantially" means to contain, as required, not larger than 50 wt% at a maximum, preferably not larger than 10 wt% and more preferably not larger than 5 wt% of additives such as a terminal stopping agent, a pigment and the like and other type of resin relative to the total of the composition.

The aromatic polyester resin-based biodegradable resin usable in the invention is not critical in type and preferably includes a polybutylene adipate/terephthalate copolymer, a polybutylene succinate/carbonate copolymer, a polyethylene succinate/polybutylene succinate/terephthalate copolymer, a polytetramethylene adipate/terephthalate copolymer, a polybutylene succinate/adipate/terephthalate copolymer or the like. Other types of biodegradable resin may be further contained within a range of not impeding the effect of the composition of the invention wherein polylactic acid is present at 25 to 70 wt% of the total weight of the aromatic polyester-based biodegradable resin and polylactic acid.

In the composition made of polylactic acid and an aromatic polyester-based biodegradable resin, the lower limit of a preferred mixing ratio of the polylactic acid differs depending on the type of target product and may not be critical in some cases. The lower limit can be at not less than 25 wt%, preferably at not less than 27 wt%, more preferably at not less than 35 wt% and much more preferably at not less than 35 wt% relative to the total weight of the polylactic acid and the aromatic polyester-based biodegradable resin. If the polylactic acid is used at less than the lower limit, the coefficient of elasticity can become small and thus, no merit of blending polylactic acid is obtained. Moreover, the upper limit of a preferred mixing ratio of polylactic acid can be at not larger than 70 wt%, preferably at not larger than 68 wt%, more preferably at not larger than 65 wt% and much more preferably at not larger than 60 wt%. If the polylactic acid exceeds the upper limit, the effect of sustaining the strength and impact resistance of the resin composition can be significantly lowered at the time of irradiation with a radiation.
Especially, in the composition containing polylactic acid and the polybutylene
adipate/terephthalate copolymer, although the lower limit of a preferred mixing ratio of the polylactic acid differs depending on the type of target product and is not critical in some cases, it can be at not less than 25 wt%, preferably at not less than 27 wt% and more preferably at not less than 30 wt%, relative to the total weight of the polylactic acid and the polybutylene adipate/terephthalate copolymer. If the polylactic acid is used at less than the lower limit, the coefficient of elasticity can become 100 MPa or below and no merit of blending the polylactic acid is obtained. In addition, the upper limit of a preferred mixing ratio of the polylactic acid is at not larger than 70 wt%, preferably at not larger than 68 wt%, more preferably at not larger than 65 wt% and much more preferably at not larger than 60 wt%. If the polylactic acid is used over the upper limit, the effect of sustaining the strength and impact resistance of the resin composition can become significantly lowered at the time of irradiation of a radiation.

The biodegradable composition of the invention may further includes, if necessary, one or two or more of a wide variety of additives including terminal stopping agents, antioxidants, pigments, softening agents, plasticizers, lubricants, antistatic agents, anticlouding agents, colorants, antioxidizing agents (age inhibitors), heat stabilizers, light stabilizers, UV absorbers and the like.

As an example of the terminal stopping agent usable in the invention, mention is made of a polycarbodiimide compound. For this, there may be used ones prepared by many methods. Fundamentally, there can be used ones prepared according to conventional methods of preparing a polycarbodiimide (U.S. Patent No. 2941956, Japanese Patent Publication No. Sho 47-33279, J. Org. Chem. 28, 2068-2075 (1963), and Chemical Review 1981, Vol. 81, No. 4, PP. 619-621).

The organic diisocyanates, which can be used as a starting material for synthesis in the preparation of the polydicarbodiimides compounds, can include, for example, aromatic diisocyanates, aliphatic diisocyanates, alicyclic diisocyanates and mixtures thereof, and mention is made specifically of 1,5-naphthalene diisocyanate, 4,4'-diphenylmethane diisocyanate, 4,4'-diphenyldimethylmethane diisocyanate, 1,3-phenylene diisocyanate, 1,4-phenylene diisocyanate, 2,4-trilene diisocyanate, 2,6-trilene diisocyanate, a mixture of 2,4-trilene diisocyanate and 2,6-trilene diisocyanate, hexamethylene diisocyanate, cyclohexane-1,4-diisocyanate, xylylene diisocyanate, isophorone diisocyanate, dicyclohexylmethane-4,4'-diisocyanate, methylcyclohexane diisocyanate, tetramethylxylylene diisocyanate, 2,6-diisopropylphenyl isocyanate, 1,3,5-triisopropylbenzene-2,4-diisocyanate and the like.

In the practice of the invention, the mixing of biodegradable resins can be effected by use of a method using melt blending in a biaxial extruder. Alternatively, a master batch of a biodegradable resin formulated preliminarily with various additives may be prepared and mixed with one or plural biodegradable resins in the course of medical device molding and molded. The molding method is not critical and includes, for example an injection molding method, an extrusion molding method, a compression molding method, a blow molding method or the like.

After or during the course of molding in given shapes, assembling and packaging, the medical device, etc. of the invention can be sterilized by irradiation with a radiation at a given dose and can thus be used as a medical device, etc. The dose of an ionizing radiation to be irradiated differs depending on the type of target product and is not particularly critical, and is generally at 5 to 100 kGy, preferably at 10 kGy to 60 kGy. The kind of irradiating radiation may include an electron beam, a γ-ray or an X-ray. In view of the ease in industrial production, an electron beam from an electron accelerator or a γ-ray from cobalt 60 is preferred. More preferably, the electron beam is used. The electron accelerator allows the beam to be irradiated to the inside of a medical device., etc. having a relatively thick portion, so that it is preferred to use a medium to high energy electron accelerator with an acceleration voltage of not less than 1 MeV. Although the irradiation atmosphere of an ionizing radiation is not critical, the irradiation may be carried out in an inert atmosphere except for air or in vacuum. Although any irradiation temperature may be used, irradiation is typically implemented at room temperature.

Although the impact resistance necessary for the medical device, etc. of the invention after radiation sterilization may differ depending on the shape of a medical device, the elongation at breakage in the tensile test can be within a range of not less than 200%, preferably not less than 220% and more preferably not less than 240%. At not less than 200%, the impact resistance is high and such a device is unlikely to suffer breakage during transport or due to drop impact and is thus excellent in function.

The strength necessary for the medical device, etc. of invention after radiation sterilization may differ depending on the shape of the medical device, etc. and the yield point stress of the tensile strength can be within a range of not less than 8 Mpa, preferably not less than 9 Mpa, more preferably not less than 10 Mpa and much more preferably not less than 10 Mpa. Over the upper limit, the strength ca be high and even a thin sheet is unlikely to suffer breakage and is functionally excellent.

The medical device, etc. of the invention includes, for example, disposable injectors, syringe containers, catheter tubes, transfusion tubes, stopper cocks, trays, nonwoven fabrics, surgical gloves, gowns, sheets, filters and the like.
The invention is particularly described and should not be construed as limited to these examples.

### Example 1

### (1) Preparation of a biodegradable resin

0.75 kg of a polybutylene adipate/terephthalate copolymer (Ecoflex, made by BASF Corporation), 0.25 kg of polylactic acid (Lacea H-100, made by Mitsui Chemicals, Inc.) and 0.02 kg of Carbodilite LA-1 (made by Nisshinbo Holdings Inc.) serving as a terminal stopper were mixed together and melt kneaded by use of a biaxial kneader (Laboplastomill, made by Toyo Seiki Co., Ltd.) at a temperature of 190°C, followed by pelletization to obtain 0.5 kg of a biodegradable resin. (2) Formation of a biodegradable sheet
The biodegradable resin obtained (1) above was compressed by use of a bench heat press machine (SA-303, made by Tester Sangyo Co., Ltd.) at a temperature of 200°C at a compression pressure of 20 MPa, followed by quenching and molding in the form of a 150 mm wide, 150 mm long and 0.5 mm thick sheet to obtain a sheet for tray.

### (3) Irradiation with a radiation

The sheet obtained in (2) above was irradiated with an electron beam of 55 kGy from a 100 MeV electron accelerator at room temperature to provide a radiation-irradiated sheet for tray.

### Example 2

In the same manner as in Example 1, a radiation-irradiated sheet for tray was made except that the starting resin in Example 1(1) was changed to a resin made of 0.70 kg of polybutylene adipate/terephthalate copolymer (Ecoflex, made by BASF Corporation), 0.30 kg of polylactic acid (Lacea H-100, made by Mitsui Chemicals, Inc.) and 0.02 kg of Carbodilite LA-1 (made by Nisshinbo Holdings Inc.).

### Example 3

In the same manner as in Example 1, a radiation-irradiated sheet for tray was made except that the starting resin in Example 1(1) was changed to a resin made of 0.60 kg of polybutylene adipate/terephthalate copolymer (Ecoflex, made by BASF Corporation), 0.40 kg of polylactic acid (Lacea H-100, made by Mitsui Chemicals, Inc.) and 0.02 kg of Carbodilite LA-1 (made by Nisshinbo Holdings Inc.).

### Example 4

In the same manner as in Example 1, a radiation-irradiated sheet for tray was made except that the starting resin in Example 1(1) was changed to a resin made of 0.50 kg of polybutylene adipate/terephthalate copolymer (Ecoflex, made by BASF Corporation), 0.50 kg of polylactic acid (Lacea H-100, made by Mitsui Chemicals, Inc.) and 0.02 kg of Carbodilite LA-1 (made by Nisshinbo Holdings Inc.).

### Example 5

In the same manner as in Example 1, a radiation-irradiated sheet for tray was made except that the starting resin in Example 1(1) was changed to a resin made of 0.40 kg of polybutylene adipate/terephthalate copolymer (Ecoflex, made by BASF Corporation), 0.60 kg of polylactic acid (Lacea H-100, made by Mitsui Chemicals, Inc.) and 0.02 kg of Carbodilite LA-1 (made by Nisshinbo Holdings Inc.).

### Example 6

In the same manner as in Example 1, a radiation-irradiated sheet for tray was made except that the starting resin in Example 1(1) was changed to a resin made of 0.30 kg of polybutylene adipate/terephthalate copolymer (Ecoflex, made by BASF Corporation), 0.70 kg of polylactic acid (Lacea H-100, made by Mitsui Chemicals, Inc.) and 0.02 kg of Carbodilite LA-1 (made by Nisshinbo Holdings Inc.).

### Example 7

In the same manner as in Example 1, a radiation-irradiated sheet for tray was made except that the starting resin in Example 1(1) was changed to a resin made of 0.40 kg of polybutylene adipate/terephthalate copolymer (Ecoflex, made by BASF Corporation) and 0.60 kg of polylactic acid (Lacea H-100, made by Mitsui Chemicals, Inc.) without addition of Carbodilite LA-1.

### (Comparative Example 1)

In the same manner as in Example 1, a radiation-irradiated sheet for tray was made except that the starting resin in Example 1(1) was changed to a resin made of 0.20 kg of polybutylene adipate/terephthalate copolymer (Ecoflex, made by BASF Corporation) and 0.80 kg of polylactic acid (Lacea H-100, made by Mitsui Chemicals, Inc.).

### (Comparative Example 2)

In the same manner as in Example 1, a radiation-irradiated sheet for tray was made except that the starting resin in Example 1(1) was changed to a resin made of 0.10 kg of polybutylene adipate/terephthalate copolymer (Ecoflex, made by BASF Corporation) and 0.90 kg of polylactic acid (Lacea H-100, made by Mitsui Chemicals, Inc.).

### (Comparative Example 3)

In the same manner as in Example 1, a radiation-irradiated sheet for tray was made except that the starting resin in Example 1(2) was changed to a polybutylene adipate/terephthalate copolymer (Ecoflex, made by BASF Corporation) alone.

### (Comparative Example 4)

In the same manner as in Example 1, a radiation-irradiated sheet for tray was made except that the starting resin in Example 1(1) was changed to a resin made of 0.30 kg of polybutylene succinate (GS Pla AZ81T, made by Mitsubishi Chemical Corporation) and 0.70 kg of polylactic acid (Lacea H-100, made by Mitsui Chemicals, Inc.).

### (Evaluation)

(Tensile test) The sheets for tray made in Examples 1 to 7 and Comparative Examples 1 to 4 were used, from which 5B-type dumbbell specimens indicated in ISO 527-2 were made by use of a punching die. Thereafter, a tensile test was carried out by use of an autograph (AG-1S, made by Shimadzu Corporation) at a testing speed of 10 mm/minute to measure a tensile yield point stress and an elongation at breakage.

The values are indicated in Table 1. It was confirmed that when polylactic acid was added to the polybutylene adipate/terephthalate copolymer, the yield point stress was improved and that when the content of polylactic acid was within a range of 25 to 70%, changes in the tensile yield point stress and elongation at breakage were small prior to and after the irradiation with radiation. Moreover, it was also confirmed that the elongation at breakage lowered when using polybutylene succinate in place of the polybutylene adipate/terephthalate.

**Table 1**

| | Yield point stress (MPa) | | | Elongation at breakage (%) | | |
|---|---|---|---|---|---|---|
| | No irradiation | After irradiation | Change rate (%) | No irradiation | After irradiation | Change rate (%) |
| Example 1 | 10 | 10 | 103 | 690 | 740 | 108 |
| Example 2 | 19 | 20 | 103 | 720 | 670 | 93 |
| Example 3 | 22 | 21 | 93 | 570 | 590 | 102 |
| Example 4 | 26 | 26 | 99 | 450 | 460 | 102 |
| Example 5 | 35 | 36 | 103 | 420 | 390 | 92 |
| Example 6 | 37 | 38 | 102 | 420 | 240 | 57 |
| Example 7 | 19 | 18 | 97 | 670 | 790 | 116 |
| Comparative Example 1 | 47 | 40 | 85 | 370 | 90 | 23 |
| Comparative Example 2 | 57 | 53 | 92 | 220 | 10 | 6 |
| Comparative Example 3 | 7 | 8 | 103 | 1310 | 1300 | 99 |
| Comparative Example 4 | 54 | 55 | 102 | 410 | 4 | 1 |

| | | | | | | |
|---|---|---|---|---|---|---|
| Results of evaluation of tensile test (irradiation with an electron beam of 55 kGy) Change rate: Value obtained by dividing a value after irradiation by a value of a non-irradiated sample. | | | | | | |

## Claims

1. An ionizing radiation-sterilized medical device or medical material, **characterized by** comprising a composition made of an aromatic polyester-based resin and polylactic acid wherein 25 to 70 wt% of said composition is polylactic acid.

2. The medical device or medical material as defined in Claim 1, wherein said aromatic polyester-based biodegradable resin is made of a polybutylene adipate/terephthalate copolymer, a polybutylene succinate/terephthalate copolymer or a copolymer thereof with polylactic acid.

3. A method for making a medical device or medical material, **characterized by** comprising molding a composition made of an aromatic polyester-based biodegradable resin and polylactic acid wherein 25-70 wt% of said composition is polylactic acid, and subjecting to irradiation with an ionizing radiation.

4. The method for making a medical device or medical material as defined in Claim 3, wherein said aromatic polyester-based biodegradable resin is made of a polybutylene adipate/terephthalate copolymer, a polybutylene succinate/terephthalate copolymer or a copolymer thereof with polylactic acid.
